# EUROPEAN PATENT APPLICATION

(11) **EP 3 808 835 A1**
(43) Date of publication of application: **21.04.2021**
(21) Application number: 19819093.6
(22) Date of filing: 13.06.2019
(51) Int. Cl.: C12M 3/02, B03B 5/62

(54) **CELL CULTURE SYSTEM AND CELL CULTURE METHOD**

(30) Priority: 13.06.2018 JP 2018112897; 13.06.2018 JP 2018112903
(71) Applicant: IHI Corporation, Koto-ku Tokyo 135-8710 (JP)
(72) Inventor: IKEDA Ryosuke, Tokyo 135-8710 (JP); ISO Yoshiyuki, Tokyo 135-8710 (JP); KAMEKURA Koichi, Tokyo 135-0061 (JP); MIZUNUMA Takato, Tokyo 135-0061 (JP); TOMIMATSU Hirofumi, Tokyo 135-0061 (JP)
(74) Representative: Grünecker Patent- und Rechtsanwälte PartG mbB
(86) International application number: PCT/JP2019/023481
(87) International publication number: WO 2019/240221

(57) **Abstract**

The cell culture system has a culture tank that houses a liquid medium for culturing cells, and a cell separation device. The cell separation device has a fluid force separation device and a liquid feed unit. The fluid force separation device has a curved flow path having a rectangular cross-section and separates relatively large cells from cells contained in the liquid medium using a vortex flow generated by flow through the curved flow path. The liquid feed unit circulates the liquid medium through the fluid force separation device in a controlled pressure environment so as to suppress any decrease in cell viability caused by pressure fluctuations in the liquid medium during circulation through the fluid force separation device.

## Description

### Technical Field

The present disclosure relates to a cell culture system and a cell culture method for efficiently obtaining useful substances through cell culture.

### Background Art

Recently, in a wide range of fields including the pharmaceutical industry, attention has been paid to the use of useful substances such as antibody substances and functional substances produced by animal cells. In order to realize the market supply of useful substances, various ingenuity and improvement have been made in the optimization and efficiency of conditions in cell culture, the isolation and purification method of the produced useful substances, and the like.

Cell culture methods are generally classified into two types: batch type and continuous type. In the batch culture method, a predetermined amount of liquid medium and cells are put into a culture tank, and when the cells grow to a certain concentration, the growth is stopped due to lack of nutrients or poisoning by metabolites. Therefore, the culture is terminated at that point. In the continuous culture method, a predetermined amount of liquid medium and cells are put into a culture tank to proliferate the cells, and at the same time, a part of the liquid medium is extracted and a new liquid medium is put into the replacement. Thus, the cell culture is continued using the supplemented nutrients. As an advantage of the continuous culture method, the culture can be continued for a long period of time and a high concentration of cultured cells can be obtained, so that improvement of productivity and equipment cost are expected. However, since the extracted liquid medium contains the cells, it is necessary to separate the cells from the extracted liquid medium and return them to the culture tank. Membrane separation or centrifugation is used as a means for separating or concentrating the cells contained in the liquid medium. However, the membrane separation is prone to clogging and cell damage. The centrifugation requires time for separation, and even if a small amount of separation at the experimental level is good, its applicability to practical use is not high.

Japanese Patent Application Laid-open No. 2017-502666 (Patent Literature 1 below) relates to an apparatus for cell culture, and describes an acoustic standing wave cell separator which communicates to the outlet of the bioreactor. In the separation by an acoustic standing wave, when applying a high frequency wave from different directions to generate a standing wave, particles are concentrated in the node of the standing wave. Utilizing this phenomenon, cells are separated from the liquid medium by concentrating the cells in the nodes of the standing wave.

On the other hand, as a document relating to the separation of particles contained in a fluid, there is Japanese Patent Application Laid-open 2016-526479 (Patent Literature 2 below), which describes a hydrodynamic separation device having a curved channel. In this device, a fluid containing particles is supplied to the curved channel, so that the force acting on the fluid flowing the curved channel can be used to separate the particles.

### Citation List

### Patent Literature

Patent Literature 1: Japanese Patent Application Laid-open No. 2017-502666
Patent Literature 2: Japanese Patent Application Laid-open No. 2016-526479

### Summary of Invention

### Technical Problem

As described above, in order to put the continuous culture of cells into practical use, a separation method for separating the cells from the liquid medium in the culture is important. In this regard, in the apparatus described in Patent Literature 1, the physical influence on the cell due to the action of high frequency waves is large. Therefore, there is a concern that the viability of cells after separation may decrease, and even if the separated cells are used to repeat the culture, the culture efficiency may decrease due to the decrease in the viability.

Since the separation technique described in Patent Literature 2 relates to separation of solid particles, it can be expected that it will be simply used when separating cells after the completion of culturing. However, if applied to the cell culture, it is necessary to examine the effect on the cells, and in particular, when separating the cells in the middle of culture from a liquid medium, the requirements for the separation technique become more stringent.

As described, since the requirements for separating the cells from the medium after culturing and the requirements for separating the cells in the middle of culturing from the liquid medium are different, it is necessary, for applying the separation technique to cell culture, to thoroughly study whether efficient cell culture is possible.

An object of the present disclosure is to provide a cell culture system and a cell culture method capable of efficiently separating cells from a medium and carrying out continuous culture in which the separated cells appropriately maintain their proliferative ability.

### Technical Solution

In order to solve the above problem, the effects on cells when separating the cells from the medium have been examined, and it has been found possible to efficiently concentrate and separate the cells from the medium and continue culturing by using the hydrodynamic separation technique.

According to an aspect of the present disclosure, a subject of a cell culture system is to comprise a culture tank that contains a liquid medium that cultures cells, and a cell separator, the cell separator comprising: a hydrodynamic separation device having a curved flow channel having a rectangular cross-section, and separating relatively large cells from the cells contained in the liquid medium using a vortex flow generated by flow through the curved flow channel; and a liquid feeding unit which flows the liquid medium through the hydrodynamic separation device in a pressure environment controlled so as to suppress decrease in cell viability caused by pressure fluctuation in the liquid medium during flowing through the hydrodynamic separation device.

According to another aspect of the present disclosure, a subject of a cell culture system is to comprise a culture tank that contains a liquid medium that cultures cells, and a cell separator, the cell separator comprising: a hydrodynamic separation device having a curved flow channel having a rectangular cross-section, to separate relatively large cells from the cells contained in the liquid medium, using a vortex flow generated by flow through the curved flow channel; and a liquid feeding unit which flows the liquid medium through the hydrodynamic separation device so as to suppress decrease in cell viability in the liquid medium during flowing through the hydrodynamic separation device.

The hydrodynamic separation device may be configured to have a single inlet for taking in the liquid medium and at least two outlets for discharging the separated liquid medium, wherein a liquid medium containing the relatively large cells concentrated is discharged from one of the at least two outlets, and the rest of the liquid medium containing relatively small cells is discharged from the other of the at least two outlets. The liquid feeding unit has an urging device that urges the liquid medium with a flow pressure for supplying the liquid medium to the hydrodynamic separation device. The liquid feeding unit has a pressure control mechanism that controls the pressure environment so that a pressure difference between the liquid medium introduced to the hydrodynamic separation device and the liquid medium derived from the hydrodynamic separation device is equal to or less than a predetermined value. The pressure control keeps high the viability of the cells.

The pressure control mechanism may comprise: a pressure monitoring unit to monitor a pressure of the liquid medium supplied to the hydrodynamic separation device; and a pressure adjusting member that adjusts the pressure of the liquid medium supplied to the hydrodynamic separation device, based on the pressure monitored by the pressure monitoring unit. The liquid feeding unit may further have a flow rate-controlling mechanism for controlling a flow rate of the liquid medium supplied to the hydrodynamic separation device. The flow rate-controlling mechanism comprises: a flow meter to monitor the flow rate of the liquid medium supplied to the hydrodynamic separation device; and a flow rate-adjusting member to adjust the flow rate of the liquid medium supplied to the hydrodynamic separation device, based on the flow rate monitored by the flow meter, and the flow rate of the liquid medium supplied to the hydrodynamic separation device is suitably adjusted according to a size of the cells to be separated.

The cell culture system further comprises: a circulation system which returns a liquid medium containing the relatively large cells separated by the hydrodynamic separation device of the cell separator to the culture tank, to circulate the liquid medium between the culture tank and the cell separator. The cell culture system further comprises: a medium supplement unit which replenishes the culture tank with a new liquid medium corresponding to an amount of the rest of the liquid medium. Thus, the cell culture can be continuously performed. The medium supplement unit comprises: a monitor that monitors an amount of the new liquid medium replenished in the culture tank; and a flow rate-adjusting member that adjusts a flow rate of the new liquid medium replenished to the culture tank, based on the amount monitored by the monitor. The above monitoring can be performed using a flow meter, a liquid level meter, a weight scale, or the like.

The cell separator may be configured to further comprise: a temporary container for accommodating the rest of the liquid medium discharged from the other outlet of the hydrodynamic separation device; an additional hydrodynamic separation device; and an additional liquid feeding unit. The additional liquid feeding unit is capable of flowing the liquid medium contained in the temporary container through the additional hydrodynamic separation device in a pressure environment controlled so as to suppress decrease in cell viability caused by pressure fluctuation in the liquid medium during flowing through the additional hydrodynamic separation device. Moreover, the additional liquid feeding unit may have an urging device that urges the liquid medium with a flow pressure for supplying the liquid medium to the additional hydrodynamic separation device. The urging device can be configured to comprise: a pump that urges the liquid medium at a connection path connecting the temporary container and the additional hydrodynamic separation device; or a pressurizing device that pressurizes the inside of the temporary container and supplies the flow pressure to the contained liquid medium.

Alternatively, the cell separator may further comprise: a temporary container for accommodating the rest of the liquid medium discharged from the other outlet of the hydrodynamic separation device; a return path for resupplying the rest of the liquid medium contained in the temporary container to the hydrodynamic separation device; and a switching mechanism for switching between supply of the liquid medium from the culture tank to the hydrodynamic separation device and supply of the rest of the liquid medium from the return path to the hydrodynamic separation device. Such a configuration is possible by switching of the switching mechanism that the liquid medium of the culture tank and the rest of the liquid medium of the temporary container are alternately supplied to the hydrodynamic separation device. The liquid feeding unit includes: a supply path connecting the culture tank and the hydrodynamic separation device; and an urging device that urges the liquid medium with a flow pressure for supplying the liquid medium to the hydrodynamic separation device, and the return path may be connected to join the supply path. The urging device comprises: a pump that urges the liquid medium at the supply path; or a pressurizing container that temporarily stores the liquid medium in the supply path and pressurizes the stored liquid medium to apply the flow pressure.

Alternatively, such a configuration is possible that the liquid feeding unit includes: a supply path connecting the culture tank and the hydrodynamic separation device; and a pressurizing container that temporarily stores the liquid medium in the supply path and pressurizes the stored liquid medium to apply a flow pressure for supplying the liquid medium to the hydrodynamic separation device. In that case, the cell separator may further comprise: a temporary container for accommodating the rest of the liquid medium discharged from the other outlet of the hydrodynamic separation device; a return path that connects the downstream side of the pressurizing container in the supply path with the temporary container and resupplies the rest of the liquid medium contained in the temporary container to the hydrodynamic separation device; and a switching mechanism. The switching mechanism is capable of switching between supply of the liquid medium from the culture tank to the hydrodynamic separation device and supply of the rest of the liquid medium from the return path to the hydrodynamic separation device, wherein, by switching the switching mechanism, the liquid medium of the culture tank and the rest of the liquid medium of the temporary container are alternately supplied to the hydrodynamic separation device.

The switching mechanism may have an on-off valve or a check valve provided on each of the upstream and downstream sides of the pressuring container in the supply path, and on the return path. The cell separator may further comprise a reflux path to send the liquid medium containing the relatively large cells concentrated from one of the outlets of the hydrodynamic separation device to the culture tank. Such a configuration is possible that an exit-side end of the reflux path is connected near an entrance of the supply path so that the entrance of the supply path is also possibly used as exit of the reflux path.

Moreover, according to an aspect of the present disclosure, a subject of a cell culture method is to comprise: a cell culture for culturing cells in a liquid medium; and a cell separation for separating, from the liquid medium, a liquid medium containing relatively large cells, the cell separation comprising: a separation process to separates the medium containing the relatively large cells concentrated from the liquid medium containing the cells, by using a vortex flow generated by flow through a curved flow channel having a rectangular cross-section; and a pressure control to control a pressure environment of the liquid medium that flows in the separation process, so as to suppress a decrease in cell viability caused by pressure fluctuation in the liquid medium while flowing through the curved flow channel.

The above-described cell culture method further comprises: a circulation process which returns the liquid medium containing the relatively large cells separated by the separation process to the cell culture, and circulates the liquid medium between the cell culture and the separation process. Continuous cell culture is possible by further comprising: a medium supplementation which replenishes the cell culture with a new liquid medium corresponding to an amount of the rest of the liquid medium containing relatively small cells separated in the separation process.

### Advantageous Effects of Invention

According to the present disclosure, it is possible to efficiently separate cells from a liquid medium containing cultured cells, and suitably maintain the proliferative ability of the cells after separation. Therefore, a cell culture system and a cell culture method capable of efficiently performing continuous culture are provided, and it is possible to efficiently obtain useful substances produced by cells.

### Brief Description of Drawings

[FIG. 1] FIG. 1 is a schematic configuration diagram showing an embodiment of a cell culture system.
[FIG. 2] FIG. 2 is a schematic configuration diagram showing another embodiment of the cell culture system.
[FIG. 3] FIG. 3 is a graph showing the relationship between the Dean number and the separation efficiency in cell separation by a hydrodynamic separation device.
[FIG. 4] FIG. 4 is a graph showing the relationship between pumps used in cell separation and cell viability.
[FIG. 5] FIG. 5 includes graphs for comparing culture with cell separation with batch culture, wherein FIG. 5(a) shows the relationship between the culture time (hours) and the viable cell density (× 10⁶ cells/mL), and FIG. 5(b) shows the relationship between the culture time (hours) and the average cell diameter (µm).
[FIG. 6] FIG. 6 is a graph showing the relationship between the culture time (hours) and the cell viability (%) in the culture shown in FIG. 5.
[FIG. 7] FIG. 7 is a schematic configuration diagram showing further embodiment of the cell culture system.
[FIG. 8] FIG. 8 is a schematic configuration diagram showing further embodiment of the cell culture system.
[FIG. 9] FIG. 9 is a schematic configuration diagram showing further embodiment of the cell culture system.
[FIG. 10] FIG. 10 is a schematic configuration diagram showing further embodiment of the cell culture system.
[FIG. 11] FIG. 11 is a schematic configuration diagram showing further embodiment of the cell culture system.
[FIG. 12] FIG. 12 is a schematic configuration diagram showing further embodiment of the cell culture system.
[FIG. 13] FIG. 13 is a schematic configuration diagram showing further embodiment of the cell culture system.

### Description of Embodiments

Description for embodiments of the present disclosure will follow, with reference to the accompanying drawings. Note that dimensions, materials, concrete numerical values and the like indicated in the embodiments are only examples for facilitating understanding the contents of the present disclosure and do not limit the present disclosure unless otherwise noted. Moreover, in the description and the drawings of the present disclosure, elements having substantially an identical function and configuration are shown with denoted by identical reference numerals, and overlapped description will be omitted. Elements not directly related to the present disclosure are not illustrated.

One of the separation techniques for separating particles contained in a liquid is to utilize the action of a Dean vortex generated in a fluid (see Patent Literature 2 above. Hereinafter, this technique is referred to as hydrodynamic separation). This separation technique utilizes the fact that Dean vortices occur in a liquid flowing through a curved flow channel curved to one side and having a rectangular cross section perpendicular to the flow direction, causing a bias in the distribution of particles in the liquid. The particles flowing through the curved flow channel have different distributions in the flow channel depending on their size (see Patent Literature 2). Specifically, a ring-shaped particle distribution is formed in the cross section of the flow channel, and the particles flow in the flow channel in a spiral shape. At this time, relatively large particles are located on the outside of the ring, and the relatively small particles are located on the inside. Moreover, the distribution form of the particles further changes by setting predetermined separation conditions, and the particles exceeding a certain size converge to the outer circumferential side of the flow channel.

In the present disclosure, the above-mentioned hydrodynamic separation is applied to the separation of culture cells contained in a liquid medium, and a separation form in which cells of a predetermined size or larger converge toward the outer peripheral side of the curved flow channel is used. In the culture system of the present disclosure, the liquid medium is supplied to the curved flow channel at a relatively high flow rate. While the supplied liquid medium flows through the curved flow channel, relatively small cells are distributed like drawing a ring in the cross section of the flow channel. On the other hand, relatively large cells converge so as to be ubiquitous on the outside (outer peripheral side) of the curved flow channel. Therefore, a liquid medium in which relatively large cells are concentrated can be sorted by separating into the fraction in which large cells concentrate and the remaining fraction. The large cell-enriched fraction contains a small amount of relatively small cells, but the concentration of small cells is significantly reduced compared to before the isolation. The remaining fraction contains most of the small cells, as well as many of the metabolites produced by cell culture, micro-condensates of waste products, dead cell fragments (debris), and the like. In this way, it is possible to separate relatively large particles and small particles by adjusting the separation state according to the conditions under which the liquid medium flows. The size of the cells to be concentrated can be adjusted by setting the flow velocity (flow rate) of the supplied liquid medium and the size of the cross section in the curved flow channel.

The cells grow large in the highly activity state, but when the activity decreases, the cells die and decompose in a relatively small state. Most of the relatively small cells are dead cells or dead cell fragments, and the proportion of active cells in the process of DNA synthesis is small. Therefore, by separating the fraction concentrated by relatively large cells from the liquid medium, unnecessary substances such as metabolites are removed and the amount thereof decreases. By refluxing the separated fraction of the relatively large cells to the culture tank, and by adding to the culture tank a flesh liquid medium corresponding to the remaining fraction removed, nutrients are replenished, so that cell culture can be continued. Therefore, the cell culture proceeds continuously by repeating the cell separation as described above and the reflux of the separated cells.

The hydrodynamic separation is very effective in cell separation, but separation conditions that can suppress damage to cell during the separation are prepared, in order to continue efficient cell culture. As this separation condition, the pressure environment for supplying the liquid medium to the curved flow channel is controlled so that fluctuation (pressure decrease) of the pressure applied to the cells during the separation does not exceed a certain level. The cells are resistant even under relatively high pressure, and the viability of the cells is maintained even under a pressurized supply of, for example, about 1 MPa. However, if the pressure fluctuation is large, even at a supply pressure (inlet pressure) of about 0.6 MPa, the cell damage is large and the viability decreases. Therefore, the supply of the liquid medium is controlled so that the fluctuation of the pressure applied to the cells (difference between the inlet pressure and the outlet pressure) during the separation is equal to or less than a predetermined value. Specifically, the pressure fluctuation (pressure difference) is controlled to be less than 0.6 MPa, and it is preferable to set it to 0.45 MPa or less, more preferably 0.40 MPa or less. Under the conditions of separation in which pressure fluctuations are suppressed as described above, the cell viability can be maintained at about 98 % or more. Therefore, with refluxing the concentrated and separated large cells into the culture tank, proliferation efficiency can be maintained.

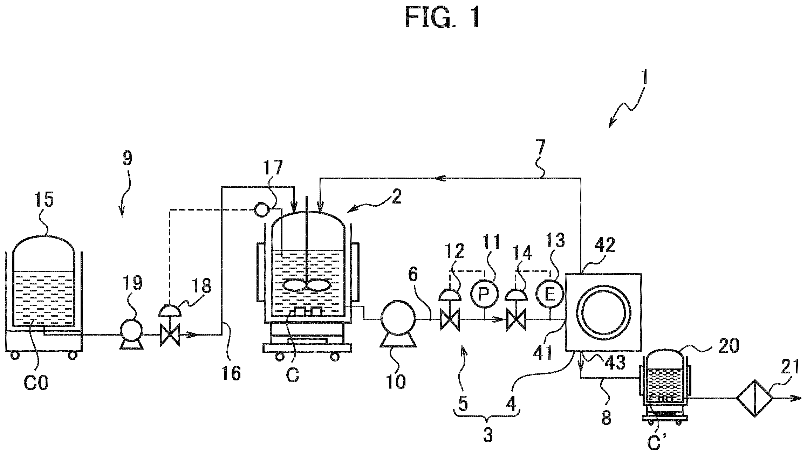

The configuration of the cell culture system will be described below with reference to one embodiment shown in FIG. 1. A cell culture system 1 of FIG. 1 has a culture tank 2 that contains a liquid medium for culturing cells, and a cell separator 3. The cell separator 3 has a hydrodynamic separation device 4 and a liquid feeding unit 5, and the liquid medium C in the culture tank 2 is supplied to the hydrodynamic separation device 4 through the liquid feeding unit 5. The hydrodynamic separation device 4 has, therein, a curved flow channel having a constant rectangular cross-section perpendicular to the flow direction. It has a single inlet 41 for taking in the liquid medium C at one end of the curved flow channel, and at least two outlets 42 and 43 for separating and discharging the liquid medium at the other end of the curved flow channel. The hydrodynamic separation device 4 utilizes a vortex flow generated in the liquid swirling in one direction by flowing through the curved flow channel, to move relatively large cells of the cells contained in the liquid medium C and make them ubiquitous on the outside (outer circumferential side) of the flow channel. Therefore, by dividing the liquid medium discharged from the curved flow channel into an outer fraction and an inner fraction, a liquid medium in which the relatively large cells are concentrated can be separated as the outer fraction. The liquid medium containing the relatively large cells concentrated is discharged from one outlet 42, and the rest of the liquid medium containing relatively small cells is discharged from the other outlet 43. The curving shape of the curved flow channel includes a substantially circumferential shape, a substantially arc (partial circumference) shape, a spiral shape, and the like, and any of these shapes may be used. The hydrodynamic separation device 4 can be designed with a flow channel unit having one curved flow channel as a constituent unit. Specifically, as a flow channel unit, a flat layer-shaped molded body in which one curved flow channel is formed therein is formed of plastic or the like. At that time, it is configured so that both ends of the curved flow channel open to the end face of the molded body to have one inlet and at least two outlets. Using such a molded body as a flow channel unit, a hydrodynamic separation device can be configured by one flow channel unit or a combination of a plurality of flow channel units. By stacking a plurality of flow channel units to form a parallel-shaped flow channel, the processing flow rate of the liquid medium can be increased.

The liquid feeding unit 5 has a supply path 6 composed of a pipe connecting the culture tank 2 and the hydrodynamic separation device 4, and the liquid medium C containing the cells of the culture tank 2 is sent to the hydrodynamic separation device 4 through the supply path 6. The cell culture system 1 of FIG. 1 has a pipe constituting a reflux path 7, and the reflux path 7 connects the hydrodynamic separation device 4 of the cell separator 3 and the culture tank 2 so that the liquid medium can be circulated between the culture tank 2 and the cell separator 3. That is, the cell culture system 1 has a circulation system composed of the supply path 6 and the reflux path 7.

The fraction of the liquid medium containing the relatively large cells separated by the hydrodynamic separation device 4 of the cell separator 3 is returned to the culture tank 2 through the reflux path 7, and the cell culture is further continued. A fraction of the remaining liquid medium C' (fraction on the inner circumferential side) containing the relatively small cells is discharged from the hydrodynamic separation device 4 through a recovery path 8. Further, in order to replenish the culture tank 2 with a new liquid medium C0 corresponding to the amount of the remaining liquid medium C' discharged from the hydrodynamic separation device 4, a medium supplement unit 9 is provided. Then the liquid medium C in the culture tank 2 is maintained at a constant amount by replenishing the new liquid medium C0.

The cell separation efficiency in the hydrodynamic separation device 4 varies depending on the Dean number and the pressure in the liquid supplied to the curved flow channel, and there are appropriate ranges of the Dean number and the pressure capable of suitable separation. The Dean number is expressed by the formula: De = Re (D/2Rc)^{1/2} (Re: Reynolds number (-), D: representative length (m), Rc: turning radius of the flow channel (m)). Since the Dean number is proportional to the flow rate of the liquid, suitable cell separation is carried out by appropriately controlling the flow rate and pressure of the liquid supplied to the hydrodynamic separation device. In general, the Dean number is preferably 30 or more and 100 or less, and more preferably about 50 to 80. Therefore, the flow velocity (flow rate) of the liquid medium is set so that the Dean number is in such a range.

The liquid feeding unit 5 of the cell culture system 1 has an urging device for urging the liquid medium C with a flow pressure for supplying the liquid medium C to the hydrodynamic separation device 4, specifically, a pump 10. The flow rate and flow pressure of the liquid medium C supplied to the hydrodynamic separation device 4 change depending on the flow pressure applied by the pump 10. Cell viability is an important factor in returning the isolated relatively large cells to the culture tank 2 to continue cell culture. In this regard, it has been found that the viability of cells during separation in the hydrodynamic separation device 4 varies depending on the magnitude of pressure fluctuation. That is, in order to prevent a decrease in the viability, it is effective to reduce the pressure fluctuation in cell separation. Therefore, on the basis of this point, the pressure environment in which the liquid feeding unit 5 flows the liquid medium C through the hydrodynamic separation device 4 is controlled so as to suppress the decrease in cell viability caused by pressure fluctuation in the liquid medium while flowing through the hydrodynamic separation device 4.

That is, the liquid feeding unit 5 has a pressure control mechanism, whereby the pressure environment is controlled so that the pressure difference between the liquid medium introduced to the hydrodynamic separation device 4 and the liquid medium derived from the hydrodynamic separation device 4 becomes equal to or less than a predetermined value. The pressure control mechanism can be configured by: a pressure monitoring unit to monitor a pressure of the liquid medium supplied to the hydrodynamic separation device 4; and a pressure adjusting member to adjust the pressure of the liquid medium supplied to the hydrodynamic separation device 4, based on the pressure monitored by the pressure monitoring unit. Specifically, in the cell culture system 1 of FIG. 1, a pressure gauge 11 is provided as the pressure monitoring unit, and a pressure regulating valve 12 is provided on the supply path 6 as the pressure adjusting member. In this cell culture system 1, the pressure on the exit side at the outlets 42 and 43 of the hydrodynamic separation device 4 is released to atmospheric pressure. Therefore, the pressure difference between the introduced liquid medium and the derived liquid medium is equal to the pressure (gauge pressure) measured by the pressure gauge 11. Therefore, pressure control can be performed based on this measured value. The pressure environment is controlled so that the pressure difference is less than 0.60 MPa, in order to prevent the decrease in viability in the cells being separated. It is preferably controlled to be 0.45 MPa or less, more preferably 0.40 MPa or less.

As described above, the separation efficiency in the hydrodynamic separation device 4 depends on the flow velocity of the liquid medium flowing through the curved flow channel. Therefore, the liquid feeding unit 5 further has a flow rate-controlling mechanism for controlling a flow rate of the liquid medium supplied to the hydrodynamic separation device 4. The flow rate of the liquid medium flowing through the supply path 6 is controlled so that the liquid medium flowing through the curved flow channel of the hydrodynamic separation device 4 has an appropriate flow rate. A flow meter 13 and a flow rate-adjusting valve 14 configure the flow rate-controlling mechanism. The flow meter 13 monitors the flow rate of the liquid medium C supplied to the hydrodynamic separation device 4, and the flow rate-adjusting valve 14 functions as a flow rate-adjusting member that adjusts the flow rate of the liquid medium C supplied to the hydrodynamic separation device 4, based on the flow rate monitored by the flow meter 13. The flow rate of the liquid medium supplied to the hydrodynamic separation device 4 is adjusted according to the size of the cells to be separated.

In the cell culture system 1, if it is possible to adjust appropriately the flow rate of the liquid medium supplied to the hydrodynamic separation device 4 by means of drive control of the pump 10, the flow rate-adjusting valve 14 may be omitted. Further, when the supply pressure of the liquid medium supplied to the hydrodynamic separation device 4 can be adjusted appropriately by the drive control of the pump 10, the pressure regulating valve 12 can be omitted. Therefore, the configuration of the cell culture system is possibly simplified by appropriately designing the dimensions of the supply path 6 and the reflux path 7 based on the processing capacity (size of the cross section of the flow channel and the number of flow channels) in the hydrodynamic separation device 4.

Cells are resistant to a certain amount of static pressure as long as the pressure fluctuation in the hydrodynamic separation device 4 is in the above-described range. That is, if the pressure in the liquid medium discharged from the hydrodynamic separation device 4 is high, the upper limit of the pressure range applicable to the liquid medium introduced into the hydrodynamic separation device 4 becomes high. Therefore, even for the condition setting that the pressure applied to the liquid medium introduced into the hydrodynamic separation device 4 is set to 0.6 MPa or more, dealing with it is possible by reducing the pressure difference between the introduction and the discharge of the liquid medium. That is, pressure-regulating valves may be provided in the reflux path 7 and the recovery path 8 to increase the outlet pressure in the liquid medium discharged from the outlets 42 and 43 of the hydrodynamic separation device and reduce the pressure difference. In this case, it is preferable to install pressure gauges in the reflux path 7 and the recovery path 8 to monitor the discharge pressure, so as to obtain an appropriate pressure difference. At this time, it is desirable to confirm the pressure environment so that the liquid medium flowing through the reflux path 7 does not undergo a sudden pressure fluctuation.

The medium supplement unit 9 of the cell culture system 1 has a medium tank 15 for accommodating the new liquid medium C0 and a replenishment path 16 that connects the medium tank 15 and the culture tank 2. The culture tank 2 is replenished with a new liquid medium C0 in the medium tank 15 to maintain a constant amount of the liquid medium in the culture tank 2. That is, the new liquid medium C0 corresponding to the amount of the remaining liquid medium C' divided (the fraction on the inner circumferential side) is replenished from the medium tank 15 through the replenishment path 16. For this purpose, the medium supplement unit 9 has a monitor that monitors an amount of the new liquid medium C0 replenished in the culture tank 2, and a flow rate-adjusting member that adjusts a flow rate of the new liquid medium replenished in the culture tank 2, based on the amount monitored by the monitor. In the cell culture system 1 of FIG. 1, a liquid level meter 17 is provided as the monitor, which is installed in the culture tank 2, and a flow rate-adjusting valve 18 is installed in the replenishment path 16 as the flow rate-adjusting member. The flow rate-adjusting valve 18 is controlled according to the liquid level detected by the liquid level meter 17, and the amount of liquid medium C0 supplied by a tubing pump 19 attached to the medium tank 15 is adjusted so that the liquid level of the liquid medium in the culture tank 2 is kept constant. The amount of liquid medium refluxed from the hydrodynamic separation device 4 to the culture tank 2 is reduced by the fraction of the remaining liquid medium containing the relatively small cells divided in the hydrodynamic separation device 4. Therefore, by maintaining the liquid level in the culture tank 2, replenishment corresponding to the fraction of the remaining liquid medium containing relatively small cells is performed. Such replenishment can be performed also by using a weigh scale for measuring the weight of the culture tank 2 instead of the liquid level meter, and a new liquid medium may be replenished so that the weight is kept constant. Alternatively, such replenishment may be performed by using a measuring device (flow meter) for measuring the mount of the liquid medium containing the relatively large cells refluxed to the culture tank 2 or the amount of the remaining liquid medium containing the relatively small cells. Specifically, a flow meter can be installed in the reflux path 7 or the recovery path 8 to supply the new liquid medium based on the measured value.

A recovery tank 20 is connected to the recovery path 8 to accommodate the liquid medium containing relatively small cells, debris, and the like. In this liquid medium, debris and aggregates are removed by a filter 21, and then useful components can be recovered from the liquid medium by a purification treatment. As the filter 21, a filter having an appropriate pore size may be selected according to the object to be removed, and examples thereof include a microfiltration membrane and an ultrafiltration membrane.

The culture tank 2, the medium tank 15, and the recovery tank 20 are containers capable of preventing microbial contamination, each of which is equipped with a heater or cooler and a temperature control function, and the liquid medium inside is maintained at a temperature suitable for cell culture or storage. The culture tank 2 is provided with a stirrer capable of stirring at an appropriate speed that does not damage the cells, and homogenizes the liquid medium. In addition, if necessary, those having a function of adjusting the amounts of oxygen/carbon dioxide/air, pH, conductivity, light amount, etc. can be appropriately used so that the culture environment can be adjusted to be suitable for the cells to be cultured.

As described above, in the cull culture system, the pressure environment of the liquid medium supplied to the hydrodynamic separation device 4 is controlled in order to preferably maintain the viability of the cells. Since the viability of cells is also affected by the pump 10 that supplies the flow pressure to the liquid medium containing the cells, it is preferable to select a suitable liquid feeding means as the pump 10 in order to maintain the cell viability. In order to suppress the influence on the viability of cells, it is preferable to use a pump of a type that does not apply shearing force to cells. Specifically, it is suitable to use a positive displacement pump that pushes out a constant volume of liquid by utilizing a volume change due to reciprocating motion or rotary motion. Examples of positive displacement pumps include reciprocating pumps such as piston pumps, plunger pumps, diaphragm pumps and wing pumps, and rotary pumps such as gear pumps, vane pumps and screw pumps. As an embodiment, it can be mentioned those that utilize a pressurizing tank equipped with a compressor. By pressurizing the liquid medium contained in the pressurizing tank with the compressor, the liquid medium can be pressure transported from the pressurizing tank to the hydrodynamic separation device.

The cell culture method that can be carried out in the cell culture system 1 as mentioned above is described below. The cell culture method includes a cell culture step of culturing cells in a liquid medium, and a cell separation step of separating, from the liquid medium, a liquid medium containing relatively large cells. The cell culture step is carried out in the culture tank 2, and the cell separation step is carried out in the cell separator 3. The cell separation includes a separation process carried out in the hydrodynamic separation device 4 and a pressure control to control a pressure environment of the liquid medium that flows in the separation process. In the separation process, a medium containing relatively large cells concentrated is separated from the liquid medium containing the cells, by using a vortex flow generated by flow through a curved flow channel having a rectangular cross-section. The pressure control is carried out so as to suppress a decrease in cell viability caused by pressure fluctuation in the liquid medium while flowing through the curved flow channel.

In the separation process, the liquid medium containing the cells is introduced to the curved flow channel having a rectangular cross section from a single inlet, and supplied in a uniform state to the curved flow channel. The curved flow channel of the hydrodynamic separation device is a flow channel having a rectangular cross section (radial cross section) perpendicular to the flow direction. While the uniform liquid medium flows through the curved flow channel, relatively small cells and fine particles rest on the Dean vortex and change their distribution in a circular motion in the rectangular cross section. On the other hand, for relatively large cells, the lift that stays on the outer circumferential side of the flow channel acts relatively strongly, so that the distribution is concentrated on the outer circumferential side. The terminal exit of the curved flow channel is divided into two, an outlet 42 located on the outer circumferential side and an outlet 43 on the inner circumferential side. The liquid medium containing relatively large cells concentrated is discharged from the outlet 42 on the outer circumferential side, and the rest of the liquid medium containing relatively small cells and fine particles is discharged from the outlet 43 on the inner circumferential side.

As shown in the above-described formula, the Dean number varies depending on the turning radius Rc of the curved flow channel and the cross-sectional dimension of the flow channel (the representative length D in the above formula can be regarded as the width of the curved flow channel). Therefore, the Dean number can be adjusted to a suitable value based on the design of the curved flow channel, whereby the hydrodynamic separation device is capable of carrying out concentrated separation of cells with good separation efficiency. Further, the flow rate of the fluid can be adjusted by setting either the width (radial direction) or the height of the cross section of the curved flow channel. Thus, the hydrodynamic separation device can be configured, based on the design of the curved flow channel, so that the cell separation process can be performed at an appropriate pressure and a desired flow rate. Therefore, the design of the curved flow channel can be appropriately changed so as to enable suitable separation according to the conditions of the separation target (cell size distribution, medium viscosity, etc.). From the viewpoint of cell separation efficiency, it is suited that the curved flow channel has a rectangular cross section having an aspect ratio (width/height) of 10 or more. By supplying the liquid medium to such a curved flow channel at a flow rate of approximately 10 to 500 mL/min, the separation proceeds satisfactorily and the cell separation process can be performed with an efficiency of about 5 to 25 billion cells/min. As relatively large cells, cells having a particle size of about 70 µm or more can be concentrated and fractionated as a fraction on the outer circumferential side, and most of the relatively small cells of about 20 µm or less distributed in a ring shape can be separated as a fraction on the inner circumferential side. By designing the terminal exit of the curved flow channel (dividing position of the outlets), the size and separation accuracy of the cells contained in the outer circumferential fraction can be adjusted, and it is also possible to make the lower limit of the size of the cells contained in the outer circumferential fraction smaller than 70 µm. Large cells have higher viability and activity than small cells, and dead cells and cell debris can be reduced by fractionating the outer circumferential fraction. Therefore, when the liquid medium containing relatively large cells separated by the separation process is returned to the cell culture in the culture tank and a circulation process is performed in which the liquid medium is circulated between the cell culture and the separation process, the cell culture efficiency can be increased. At this time, the amount of the liquid medium refluxed decreases by the amount of the remainder liquid medium (fraction on the inner circumferential side) containing the relatively small cells separated in the separation process. Therefore, a new liquid medium corresponding to this amount is added to the culture tank and a medium supplement is performed to replenish the liquid medium in the cell culture. This continuously replenishes the nutrients used by the cells and dilutes the concentration of metabolites, allowing continuous culturing.

The state of separated cells (size of cells to be fractionated, fractionated ratio) in the hydrodynamic separation device differs depending on the dividing position of the outlets at the exit. In general, when the cross-sectional area ratio at the flow channel exit is designed so that the division ratio (volume ratio) of the outer circumferential-side fraction/inner circumferential-side fraction is about 90/10 to 50/50, it is suitable for concentrated separation of cells as described above. In the cell culture system of FIG. 1. The hydrodynamic separation device has two outlets as the exit of the curved flow channel, but it is also possible to divide into three or more outlets. When the terminal exit is divided into three or more outlets, the system may be configured so that the amount of the fraction to be refluxed to the culture tank can be changed depending on the situation.

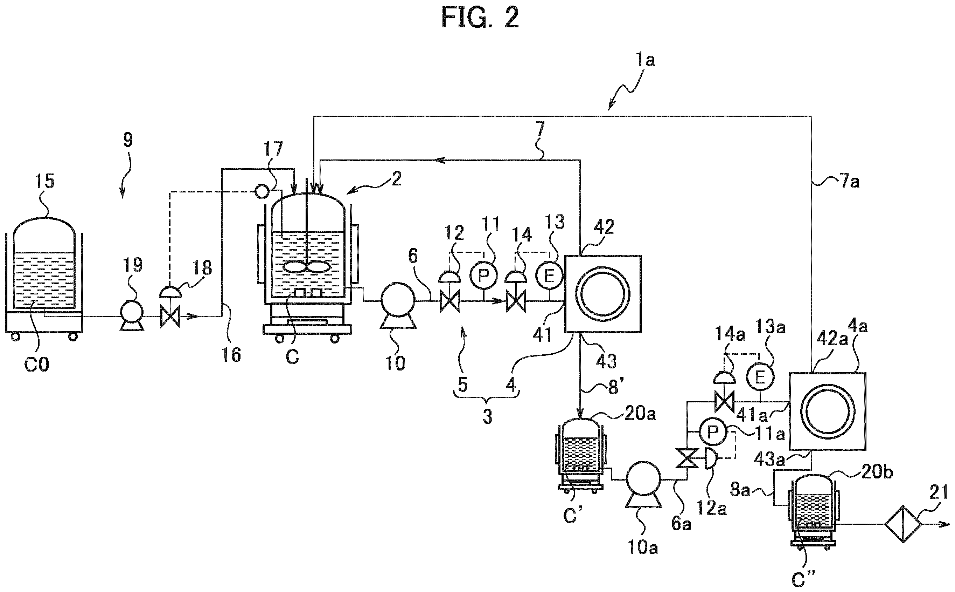

It is possible to modify the cell culture system to use a plurality of hydrodynamic separation devices as in the embodiment shown in FIG. 2. The cell culture system 1a of FIG. 2 is configured to carry out a two-step separation process using two hydrodynamic separation devices 4 and 4a. Specifically, it is configured to have a second-stage hydrodynamic separation device 4a for further separating the remaining liquid medium c' discharged from the hydrodynamic separation device 4 by the separation of the liquid medium C, that is, separating the relatively small cells contained in the fraction on the inner circumferential side. The recovery tank 20a containing the remaining liquid medium C' discharged from the first-stage hydrodynamic separation device 4 through the recovery path 8' is connected to the second-stage hydrodynamic separation device 4a by a supply path 6a. A pump 10a, a pressure gauge 11a, a pressure-regulating valve 12a, a flow meter 13a, and a flow rate-adjusting valve 14a are installed in the supply path 6a. The liquid medium C' of the recovery tank 20a is supplied to the inlet 41a of the hydrodynamic separation device 4a by the pump 10a, and the liquid medium C' is further divided into two fractions. The fraction (outer circumferential side) containing relatively large cells among those contained in the liquid medium C' is refluxed from an outlet 42a to the culture tank 2 through a reflux path 7a. The remaining liquid medium C" (fraction on the inner circumferential side) containing relatively small ones is supplied to a recovery tank 20b from an outlet 43a through a recovery path 8a. The liquid medium C" contained in the recovery tank 20b is supplied to the purification treatment through the filter 21.

In the cell culture system 1a of FIG. 2, the components contained in the liquid medium are divided into three by performing two-step cell separation. The separation conditions in the second-stage hydrodynamic separation device 4a can be adjusted by controlling the flow rate and supply pressure of the liquid medium C' supplied through the supply path 6a. By appropriately setting the separation conditions in the two hydrodynamic separation devices 4 and 4a, it is possible to improve the separation accuracy and enrichment of cells. As an example, in the cell culture system 1a of FIG. 2 configured using the culture tank 2 having a capacity of 200 L, for example, the following culture method can be carried out.

When configuring the hydrodynamic separation device 4 by using six flow channel units having a rated processing flow rate of 300 mL/min and configuring the hydrodynamic separation device 4a by using two of the same flow channel units, the separation process in the first-stage hydrodynamic separation device 4 can proceed at a flow rate of 1,800 mL/min. By this separation, the liquid medium containing relatively large cells may be refluxed to the culture tank 2 at a flow rate of 1,200 mL/min, while supplying the remaining liquid medium C' from the recovery tank 20a to the second-stage hydrodynamic separation device 4a. Then, in the second-stage hydrodynamic separation device 4a, the separation process can proceed appropriately at a flow rate of 600 mL/min. When the liquid medium on the outer circumferential side is refluxed to the culture tank 2 from the second-stage hydrodynamic separation device 4a at a flow rate of 400 mL/min, the liquid medium c" is supplied from the recovery tank 20b to the purification treatment at a flow rate of 200 mL/min. In the culture tank 2, a new liquid medium is replenished at a flow rate of 200 mL/min in order to maintain the amount of the liquid medium, and the daily medium exchange amount is to be 288 L (medium exchange rate: about 1.4 times). In this way, the concentrated relatively large cells are continuously introduced into the cell culture and nutrients are supplemented by the addition of a new liquid medium, so that the culture can be continuously advanced. Dead cells and the like are gradually removed from the liquid medium of the culture tank 2, and relatively large cultured cells increases.

In the system design of the cell culture systems 1 and 1a, since the proper processing flow rate (rated flow rate) in the hydrodynamic separation device is the basis, the cell culture system may be configured so that the capacity of the culture tank 2 and the processing flow rate of the hydrodynamic separation device are properly balanced. In the case where the liquid medium capacity of the culture tank 2 is a relatively small amount such that the medium exchange rate exceeds the appropriate value when the processing in the hydrodynamic separation device is continuously performed, the processing amount in the hydrodynamic separation device may be set so that the medium exchange rate becomes an appropriate value. Then, the processing time may be calculated based on the rated flow rate, and the separation process may be performed intermittently at regular intervals in a plurality of times.

Using the cell culture system as described above, a cell culture method can be carried out on various cells, and various useful substances such as proteins and enzymes produced by cultured cells can be recovered and used for manufacturing pharmaceutical products. Specifically, it can be applied to the culture of eukaryotic cells such as animal cells (cells of mammals, birds or insects) and fungal cells (cells of fungi such as Escherichia coli or yeast). For example, Chinese hamster ovary cells, baby hamster kidney (BHK) cells, PER.C.6 cells, myeloma cells, HER cells, etc. can be mentioned. Useful substances obtained by such cell culture include, for example, immunoglobulins (monoclonal antibody or antibody fragment), fusion proteins, insulins, growth hormones, cytokines, interferons, glucagon, albumin, lysosome enzyme, human serum albumin, HPV vaccines, blood coagulation factors, erythropoietins, antibodies such as NS0 and SP2/0. The cell culture may be carried out according to a conventional method based on the culture conditions known for each cell. The liquid medium used for cell culture may be any of synthetic medium, semi-synthetic medium, and natural medium, and a medium suitable for the cells to be cultured can be appropriately selected and used. In general, selective enrichment media or selective isolation media formulated to grow a particular bacteria species are preferably used. It may be appropriately selected and used from commercially available liquid mediums, or it may be prepared using nutrients and purified water according to a known formulation. A differential agent (pH indicator, enzyme substrate, sugar, etc.) for the purpose of inspection, a selective agent for suppressing the growth of unintended microorganisms, and the like may be added as necessary. In order to promote the hydrodynamic separation efficiently, it is preferred to use a liquid medium having a low viscosity.

When the useful substance produced by the cultured cells is contained in the liquid medium, the useful substance can be recovered by purifying the fraction on the inner circumferential side recovered from the hydrodynamic separation device. Even when the useful substance to be produced is in the cultured cells, the fraction on the inner circumferential side recovered from the hydrodynamic separation device may contain the useful substance released from the dead cells. Therefore, useful substances can be similarly recovered from the recovered fraction. However, if extracting the liquid medium in the culture tank 2 at a constant ratio and separating the cells, in parallel with the cell culture, useful substances can be efficiently recovered from the cells. At this time, the cells can be concentrated and collected by using the hydrodynamic separation device 4 of the cell culture systems 1, 1a. For this purpose, a modification is appropriate to branch the reflux path 7 of the hydrodynamic separation device 4 so that the supply destination of the liquid medium containing relatively large cells discharged from the outlet 42 can be switched from the culture tank 2. In this recovery form, any of the following methods is possible: a method of alternately performing extraction of the liquid medium containing relatively large cells and reflux of it to the culture tank 2; and a method of continuously extracting at a predetermined ratio according to the growth rate of cells in the culture tank 2.

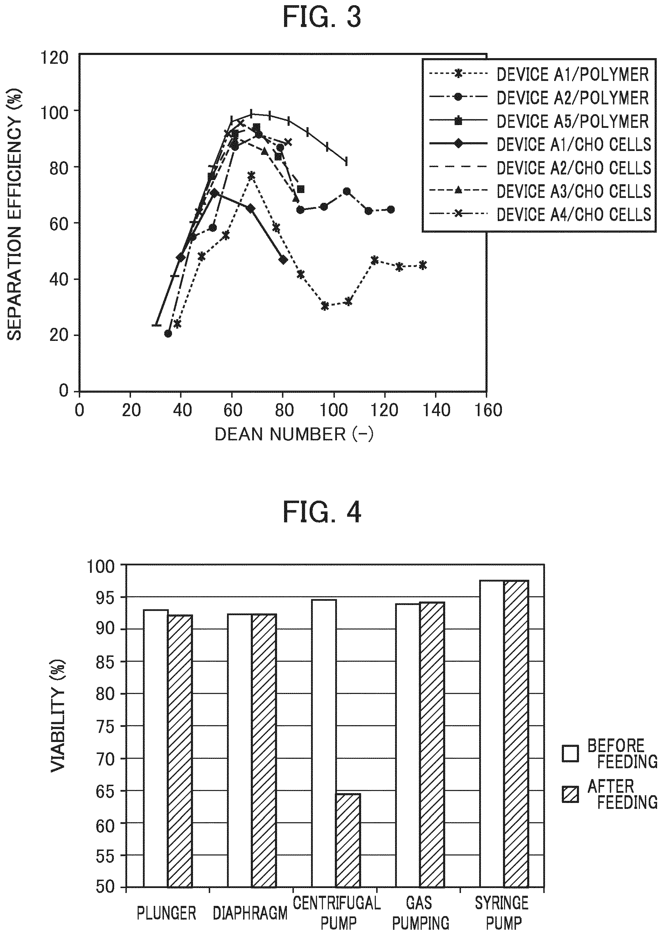

FIG. 3 is a graph showing the results of investigating the relationship between the Dean number and the separation efficiency in the hydrodynamic separation device. FIG. 3 shows the result of separation by the hydrodynamic separation device, with any of the five types of hydrodynamic separation devices (devices A1 to A5) having different flow channel dimensions, using a separation target of either polymer particles (styrenedivinylbenzene copolymer) or CHO cells (Chinese hamster ovary cells). The average particle size of each separation target is in the range of 14 to 18 µm. The separation efficiency is a value calculated as [1 - (x/X)] × 100 (%). In the calculation formula, X indicates the concentration of the separation target contained in the liquid before separation, and x is the concentration of the separation target contained in the fraction on the inner circumferential side after separation. Since the particle size distribution of each of the separation targets is narrow, the separation efficiency is based on such evaluation that the separation efficiency in a state where the entire amount of particles or cells is concentrated in the outer circumferential fraction is regarded as 100 %. As can be seen from the graph, in both polymer particles and animal cells, the separation efficiency is highest when the Dean number is around 70, and generally, high separation efficiency can be achieved under conditions where the Dean number is in the range of 50 to 80.

FIG. 4 shows the results of examining the effect on cell viability depending on the type of pump that transports the liquid medium to the hydrodynamic separation device. The liquid medium in which the cells were cultured was supplied to the hydrodynamic separation device using a pump at a discharge pressure of 0.3 MPa, and two fractions of the liquid medium discharged from the separation device were collected together and a small amount thereof was sampled. The result of measuring the viability (%, the ratio of living cells in all cells) of the sample cells with a cell measuring device (manufactured by Beckman Coulter, product name: Vi-Cell) is shown. "Gas pumping" in the graph is a form in which the liquid medium is pressure-fed with compressed air from a pressurized tank connected with a compressor, and it can be classified as a positive displacement pump. It can be seen from FIG. 4 that the centrifugal pump has a large damage to cells, and that the other pumps classified as positive displacement pumps prevent the decrease in the viability.

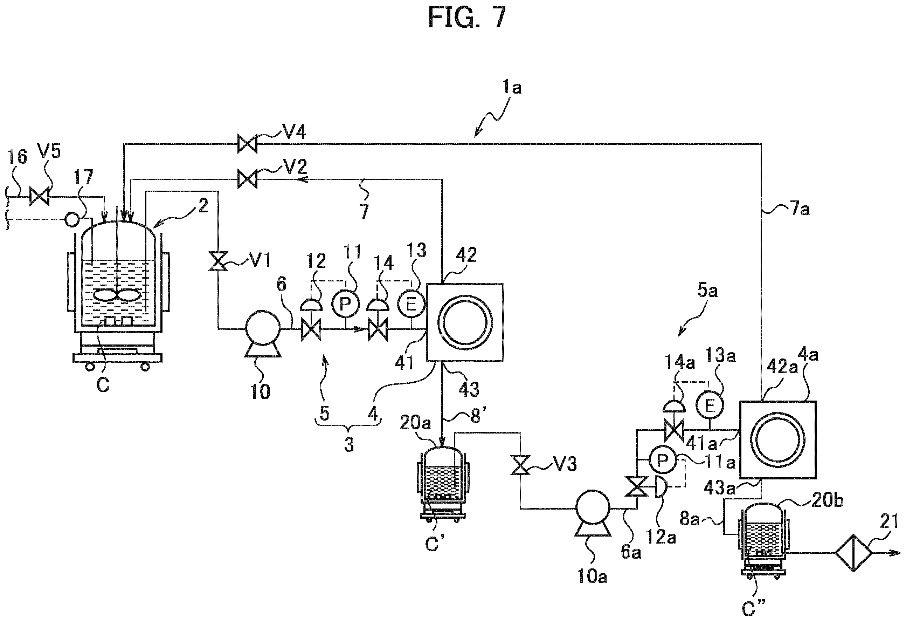

By performing the second-stage separation using an additional hydrodynamic separation device as shown in FIG. 2, suitable separation can be carried out even for a high-density cell culture medium in which proliferation has progressed. In the description of the cell culture systems of FIG. 1 and FIG. 2, the valve control that regulates the flow in the flow paths is omitted. However, in the cell culture system, an on-off valve is usually provided in the flow path and the opening/closing operation is performed at the start and end of the operation. The on-off valve for switching the connection/disconnection of the flow path will be described below with reference to FIG. 7. Regarding FIG. 7, since the medium supplement unit for replenishing the culture tank with new medium is the same as in FIG. 1 and FIG. 2, the illustration and description thereof will be omitted, and the above will be referred to.

FIG. 7 describes the installation of on-off valves in the cell culture system 1a of FIG. 2. That is, a system that performs two-step hydrodynamic separation using an additional hydrodynamic separation device 4a. An on-off valve V1 is installed in the supply path 6 for supplying the liquid medium from the culture tank 2 to the first-stage hydrodynamic separation device 4. Moreover, an on-off valve V2 is installed in the reflux path 7 for refluxing the fraction of the outer circumferential side from the outlet 42 of the hydrodynamic separation device 4 to the culture tank 2. The remaining liquid medium (fraction on the inner circumferential side) discharged from the other outlet 43 of the hydrodynamic separation device is temporarily stored in the recovery tank 20a and supplied to the hydrodynamic separation device 4a through the supply path 6a. The supply path 6a is a connection path for connecting a temporary container and the hydrodynamic separation device 4a, and an on-off valve V3 is installed therein. The pressure and flow rate of the liquid medium flowing through the supply path 6a are appropriately controlled by an additional liquid feeding unit 5a. In such a controlled pressure environment, the decrease in cell viability due to pressure fluctuation in the liquid medium is suppressed during the flow of the hydrodynamic separation device 4a. The fraction on the outer circumferential side of the liquid medium separated by the hydrodynamic separation device 4 is refluxed to the culture tank 2 through the reflux path 7a. An on-off valve V4 is installed in the reflux path 7a. Further, an on-off valve V5 is installed in the replenishment path 16 for replenishing a new liquid medium.

When starting the cell culture operation, the on-off valves V1 to V5 are opened. After the operation is stopped, it is confirmed that the liquid medium has been discharged from the flow paths to the culture tank 2 or the recovery tanks 20a and 20b, and the on-off valves V1 to V5 are then closed.

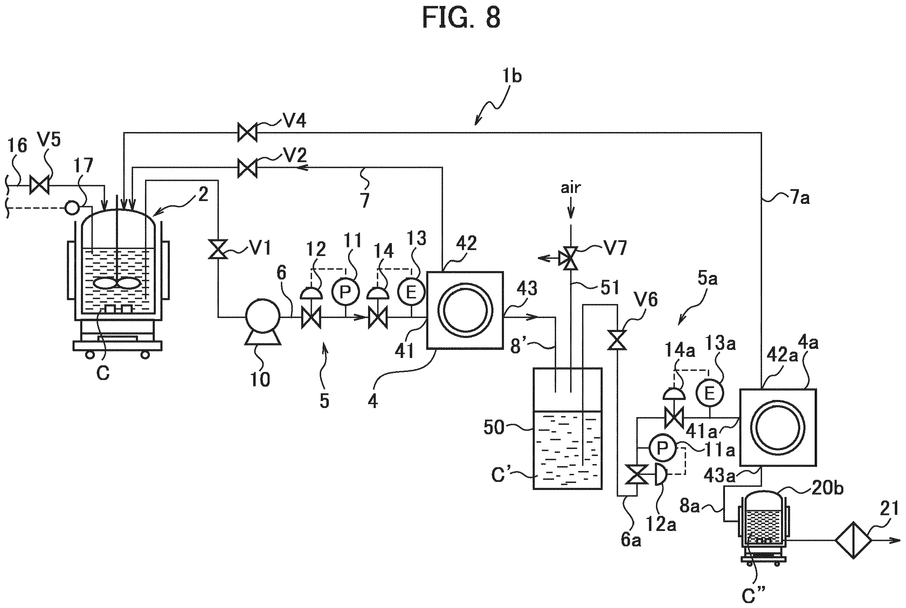

As described above, the type of pumps used as an urging device that applies flow pressure affects the viability of cells, and utilization of gas pumping using a pressure-resistant container is advantageous in preferably continuing cell culture. An embodiment of supplying the liquid medium to the hydrodynamic separation device by gas pumping will be described with reference to FIG. 8 to FIG. 10. As for FIG. 8 to FIG. 10, since the medium supplement unit for replenishing the culture tank with new medium is the same as in FIG. 1 and FIG. 2, the illustration and detailed description thereof will be omitted, and the above will be referred to.

The cell culture system 1b of FIG. 8 has a configuration in which the recovery tank 20a is used to redesign the cell culture system 1a of FIG. 7 so that gas pumping is used as an urging device instead of the pump 10a. Therefore, the pump 10a is not used. Since the other configurations are the same as those of the cell culture system 1a of FIG. 7, the description thereof will be omitted.

In FIG. 8, the remaining liquid medium C' (fraction on the inner circumferential side) discharged from the outlet 43 of the hydrodynamic separation device 4 through the recovery path 8' is accommodated in a sealed temporary container 50 of pressure resistance. The supply path 6a for supplying the liquid medium C' to the hydrodynamic separation device 4a is connected to the temporary container 50 in such a manner that its end is lower than the liquid level of the liquid medium C' stored in the temporary container 50. An on-off valve V6 is installed in the supply path 6a. A gas line 51 for supplying pressurizing air is connected to the temporary container 50, and the liquid medium to be stored is pressurized by the pressurizing air supplied. Therefore, when the on-off valve V6 is opened, the liquid medium C' of the temporary container 50 flows through the supply path 6a and is supplied to the hydrodynamic separation device 4a. The flow rate and pressure at that time are appropriately controlled by the liquid feeding unit 5a. In this way, the gas line 51 acts as a pressurizing device that pressurizes the inside of the temporary container 50 and supplies the flow pressure to the liquid medium c'. Therefore, in the recovery path 8' and the supply path 6a connecting the temporary container 50 and the hydrodynamic separation device 4a, it urges fluid pressure into the liquid medium instead of a pump. A residual pressure release valve V7 for releasing air to depressurize is installed in the gas line 51, and the liquid medium can be repeatedly delivered in the same manner as the pump by repeating pressurization and depressurization.

When the cell culture operation is started, the on-off valves V1, V2, and V4 to V6 are opened. After the operation is stopped, it is confirmed that the liquid medium has been discharged from the flow paths to the culture tank 2 or the recovery tanks 20a and 20b. Then the on-off vales V1, V2, and V4 to V6 are closed and depressurization is performed by letting the air in the gas line escape from the residual pressure release valve V7.

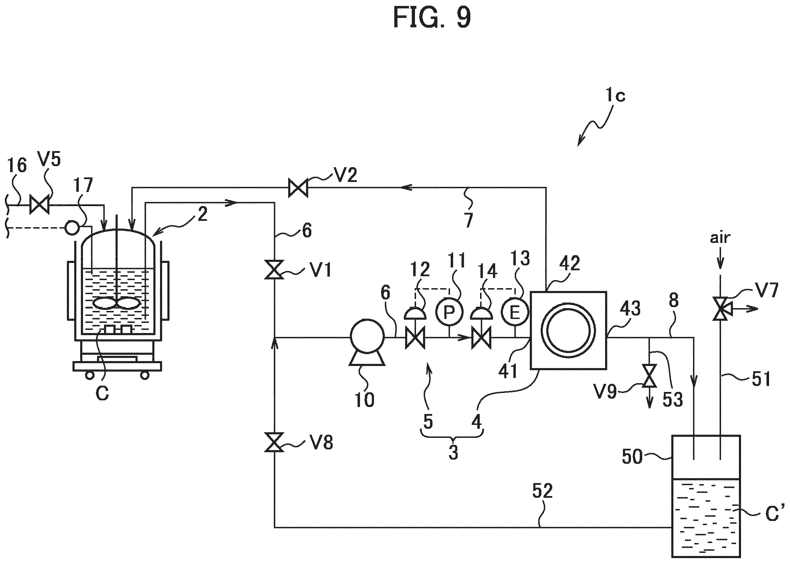

The cell culture system 1c of FIG. 9 has a configuration modified in the cell culture system 1 of FIG. 1 to perform gas pumping using the recovery tank 20. The gas pumping is configured so that the remaining liquid medium C' (fraction on the inner circumferential side) discharged from the hydrodynamic separation device 4 can be supplied again to the hydrodynamic separation device 4.

In the cell culture system 1c, an on-off valve V1 and an on-off valve V2 are installed in the supply path 6 and the reflux path, respectively, and an on-off valve V5 is installed in the replenishment path 16 for replenishing a new liquid medium. When the on-off vales V1, V2, and V5 are opened to start the operation, the liquid medium C in the culture tank 2 is supplied to the hydrodynamic separation device 4. The liquid medium (fraction on the outer circumferential side) discharged from the outlet 42 of the hydrodynamic separation device 4 is refluxed to the culture tank 2.

The cell culture system 1c has a sealed temporary container 50 of pressure resistance, instead of the recovery tank 20, and the other outlet 43 of the hydrodynamic separation device 4 is connected to the temporary container 50 by the recovery path 8. The remaining liquid medium C' (fraction on the inner circumferential side) discharged from the outlet 43 is supplied to the temporary container 50 from the recovery path 8 and is stored in the temporary container 50. Similar to FIG. 8, a gas line 51 for supplying pressurizing air is connected to the temporary container 50, and the surface of the stored liquid medium C' is pressed by the supplied pressurizing air. Further, a return path 52 is connected to the temporary container 50, and the return path 52 joins the supply path 6 on the downstream side of the on-off valve V1. An on-off valve V8 is installed on the return path 52.

Therefore, when the on-off vale V8 is opened, the liquid medium C' of the temporary container 50 returns from the return path 52 to the supply path 6 and is supplied again to the hydrodynamic separation device 4. By switching the on-off valves V1 and V8, either the liquid medium C of the culture tank 2 or the liquid medium C' of the temporary container 50 can be supplied to the hydrodynamic separation device 4. That is, the on-off valves V1 and V8 function as a switching mechanism for switching between the supply of the liquid medium from the culture tank 2 to the hydrodynamic separation device 4 and the supply of the remaining liquid medium C' from the return path 52 to the hydrodynamic separation device 4. Therefore, by switching the on-off valves V1 and V8, the liquid medium of the culture tank 2 and the remaining liquid medium C' of the temporary container can be alternately supplied to the hydrodynamic separation device 4.

In the embodiment of FIG. 9, it is possible to obtain a fraction of the inner circumferential side having a low cell density (number of cells per volume) by repeating the hydrodynamic separation, similarly to the embodiment of FIG. 2. Therefore, it is useful when the cell density of the liquid medium in the culture tank 2 is high and the cell density of the remaining liquid medium is not sufficiently reduced by one time of hydrodynamic separation. In order to collect the fraction whose cell density has decreased in this way, a discharge path 53 branching from the recovery path 8 is provided, and an on-off valve V9 is installed in the discharge path 53. Therefore, at the same time as switching the on-off valves V1 and V8, the on-off valve V9 is also switched, and the fraction whose cell density has decreased by the two times of hydrodynamic separation is intermittently collected from the discharge path 53. Instead of the on-off valves V1 and V8, a switching valve may be installed at the confluence point where the return path 52 joins the supply path, to switch the connection of the flow path.

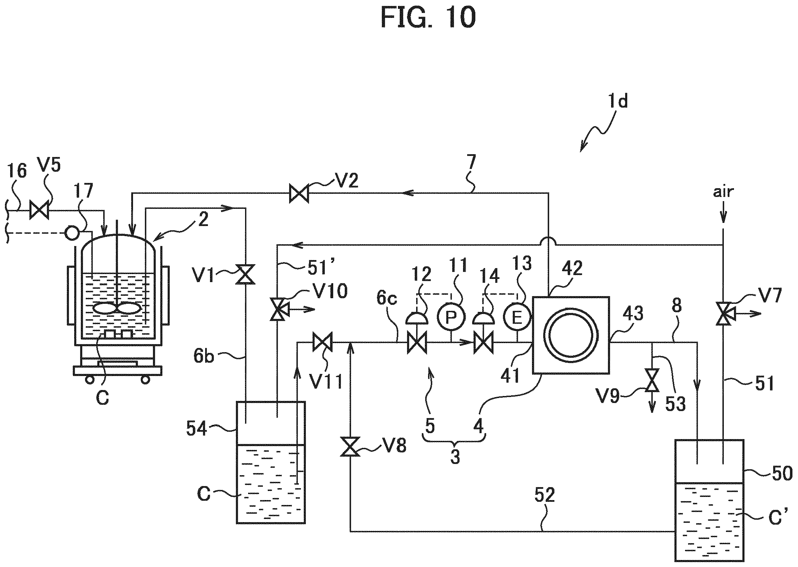

The cell culture system 1d of FIG. 10 has a configuration modified so that the cell culture system 1c of FIG. 9 use a gas line for gas pumping as an urging device, instead of the pump 10. For this purpose, a pressurizing container 54 for temporarily accommodating the liquid medium C and applying a flow pressure is installed.

Specifically, the supply path for supplying the liquid medium C of the culture tank 2 to the hydrodynamic separation device 4 is composed of a supply path 6b and a supply path 6c between which the pressurizing container 54 is interposed. An on-off valve V1 is installed in the supply path 6b on the upstream side of the pressurizing container 54, and an on-off valve V11 is installed in the supply path 6c on the downstream side. When the on-off valve V1 is opened, the liquid medium C in the culture tank 2 is supplied to the pressurizing container 54 and accommodated. A gas line 51' for supplying pressurizing air is connected to the pressurizing container 54, and the liquid medium C to be stored is pressurized by the supplied pressurizing air. Therefore, a flow pressure is applied, and the liquid medium C is supplied from the supply path 6c to the hydrodynamic separation device 4 by opening the on-off valve V11. The pressure and flow rate for supplying the liquid medium C are appropriately controlled in the liquid feeding unit 5. A residual pressure release valve V10 for letting the air escape and depressurizing is installed in the gas line 51'.

The cell culture system 1d has a temporary container 50 like the cell culture system 1c of FIG. 9. The outlet 42 of the hydrodynamic separation device 4 is connected to the culture tank 2 by the reflux path 7, and the other outlet 43 is connected to the temporary container 50 by the recovery path 8. The remaining liquid medium C' (fraction on the inner circumferential side) discharged from the outlet 43 of the hydrodynamic separation device 4 is supplied to the temporary container 50 from the recovery path 8 and is accommodated in the temporary container 50. Similar to FIG. 8, a gas line 51 for supplying pressurizing air is connected to the temporary container 50, and the liquid medium C' stored is pressurized by the supplied pressurizing air. A return path 52 is connected to the temporary container 50, and the return path 52 joins the downstream side of the on-off valve V11 and the upstream side of the liquid feeding unit 5 in the supply path 6c. An on-off valve V8 is installed on the return path 52.

Therefore, when the on-off valve V8 is opened, the liquid medium C' of the temporary container 50 is refluxed from the return path 52 to the supply path 6c and is supplied again to the hydrodynamic separation device 4. By switching the on-off valves V1 and V8, either the liquid medium C of the culture tank 2 or the liquid medium C' of the temporary container 50 can be supplied to the hydrodynamic separation device 4. Therefore, similarly to the cell culture system 1c of FIG. 9, the liquid medium of the culture tank 2 and the remaining liquid medium C' of the temporary container can be alternately supplied to the hydrodynamic separation device 4 by switching the on-off valves V11 and V8. As a result, it is possible to obtain a fraction on the inner circumferential side having a low cell density by repeating the hydrodynamic separation. Therefore, it is useful when the cell density of the liquid medium in the culture tank 2 is high and the cell density of the remaining liquid medium is not sufficiently reduced by one time of hydrodynamic separation.

In FIG. 10, the gas lines 51 and 51' for supplying the pressurizing air are branched lines having a common gas source. However, they may be two separate lines since they are individually controlled.

The multi-stage separation process in the cell culture system 1d is started by closing the on-off valves V1, V8 and V9 and opening the on-off valves V2 and V11 while the pressurizing container 54 holds the liquid medium C of the culture tank 2. Then the first hydrodynamic separation is performed. The fraction on the outer circumferential side is refluxed to the culture tank 2, and the fraction on the inner circumferential side is accommodated in the temporary container 50. The second hydrodynamic separation is started by closing the on-off valve V11 and opening the on-off valves V8 and V9. The fraction on the outer circumferential side is refluxed to the culture tank 2, and the fraction on the inner circumferential side is discharged from the discharge path 53. During standby without processing by the hydrodynamic separation device 4, the on-off valves V1, V2 and V9 are closed.

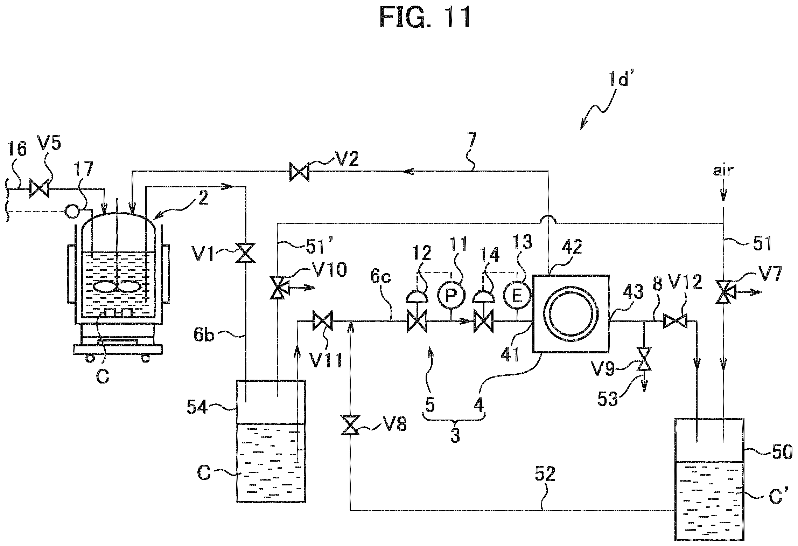

The cell culture system 1d' in FIG. 11 is provided with an on-off valve V12 on the recovery path 8 for supplying, to the temporary container 50, the remaining liquid medium C' (fraction on the inner circumferential side) discharged from the outlet 43 of the hydrodynamic separation device 4. Since the other configurations are the same as those of the cell culture system 1d of FIG. 10, the description thereof will be omitted. Further, since the medium supplement unit for replenishing the culture tank with new medium is the same as in FIG. 1 and FIG. 2, the illustration and description thereof will be omitted, and the above will be referred to.

In the cell culture system 1d' of FIG. 11, the separation processes of the two hydrodynamic separation devices 4 and 4a of the cell culture system 1a of FIG. 2 can be alternately performed in the same manner as the cell culture system 1d of FIG. 10. As a result, the liquid medium C' and the liquid medium C" as shown in FIG. 2 are alternately discharged from the outlet 43 of the hydrodynamic separation device 4 in FIG. 11. An on-off valve V9 is installed in the discharge path 53 branching from the recovery path 8, and an on-off valve V12 is installed in the recovery path 8. Therefore, the on-off valve V9 is switched at the same time as the on-off valves V1, V11 and V12 are switched, and the fraction whose cell density has decreased by the two hydrodynamic separations is intermittently collected from the discharge path 53. The on-off valve V12 prevents the remaining liquid medium C" discharged by the second separation in the hydrodynamic separation device 4 from being supplied to the primary container 50, and ensures recovery from the discharge path 53. Instead of the on-off valves V8 and V11, a switching valve may be installed at the confluence point where the return path 52 merges with the supply path 6c, to switch the connection of the flow path.

Also in the cell culture system 1d', a fraction on the inner circumferential side having a low cell density can be obtained by repeating the hydrodynamic separation using a single hydrodynamic separation device 4. Therefore, as in the cell culture system 1a of FIG. 2, it is useful when the cell density of the liquid medium in the culture tank 2 is high and the cell density of the remaining liquid medium is not sufficiently reduced by one time of hydrodynamic separation.

The multi-stage separation process in the cell culture system 1d' is started by closing the on-off valves V1, V8 and V9 and opening the on-off valves V2, V11 and V12 while the pressurizing container 54 holds the liquid medium C of the culture tank 2. Then the first hydrodynamic separation is performed. The fraction on the outer circumferential side is refluxed to the culture tank 2, and the fraction on the inner circumferential side is accommodated in the temporary container 50. The second hydrodynamic separation is started by closing the on-off valves V11 and V12 and opening the on-off valves V8 and V9. The fraction on the outer circumferential side is refluxed to the culture tank 2, and the fraction on the inner circumferential side is discharged from the discharge path 53. During standby without processing by the hydrodynamic separation device 4, the on-off valves V1, V2 and V9 are closed.

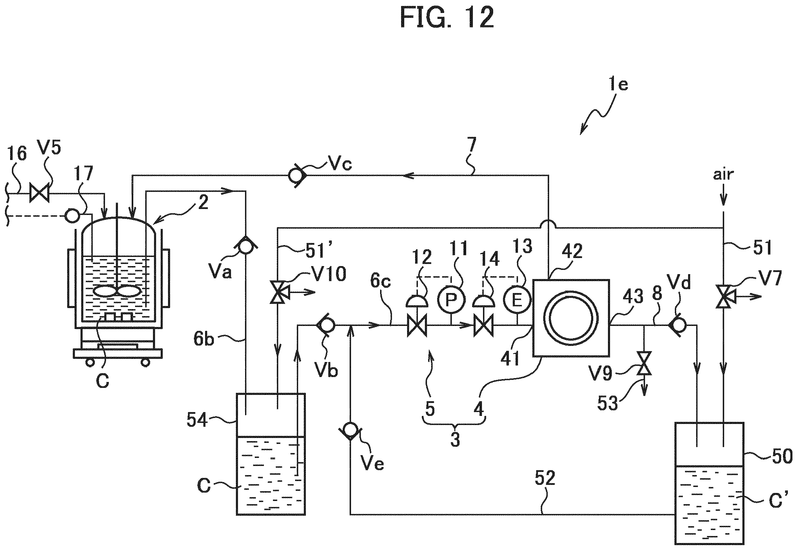

The cell culture system 1e of FIG. 12 shows an embodiment in which the on-off valves V1, V2, V8, V11 and V12 in the cell culture system 1d' of FIG. 11 are changed to check valves Va to Ve. This enables to reduce the complexity of the valve control operation.

Specifically, a check valve Va is installed in the supply path 6b to prevent backflow from the pressurizing container 54 to the culture tank 2. A check valve Vb is installed in the supply path 6c to prevent the inflow from the hydrodynamic separation device 4 and the return path 52 into the pressurizing container 54. The return path 7 has a check valve Vc installed therein to prevent the flow from the culture tank 2 side to the hydrodynamic separation device 4 side. Therefore, the circulation of the liquid medium between the culture tank 2 and the hydrodynamic separation device 4 is determined in one direction. A check valve Vd is installed in the recovery path 8, and the supply destination of the liquid medium discharged from the outlet 43 of the hydrodynamic separation device 4 can be switched only by opening and closing the on-off valve V9. In the return path 52, a check valve Ve is installed to prevent inflow from the pressurizing container 54 and the hydrodynamic separation device 4 into the return path 52.

Therefore, in the cell culture system 1c, the on-off valve V9 is closed and the separation process is started by gas pumping from the gas line 51'. The liquid medium in the culture tank 2 is supplied to the hydrodynamic separation device 4 via the pressurizing container 54 and separated. The fraction on the outer circumferential side is refluxed to the culture tank 2, and faction of the inner circumferential side is accommodated in the temporary container 50. Next, when the on-off valve V9 is opened and gas pressure feeding is performed from the gas line 51, the liquid medium C' of the temporary container 50 is supplied from the return path 52 to the hydrodynamic separation device 4 and separated. The fraction on the outer circumferential side is refluxed to the culture tank 2, and the fraction on the inner circumferential side is discharged from the discharge path 53 and does not return to the temporary container 50 being pressurized.

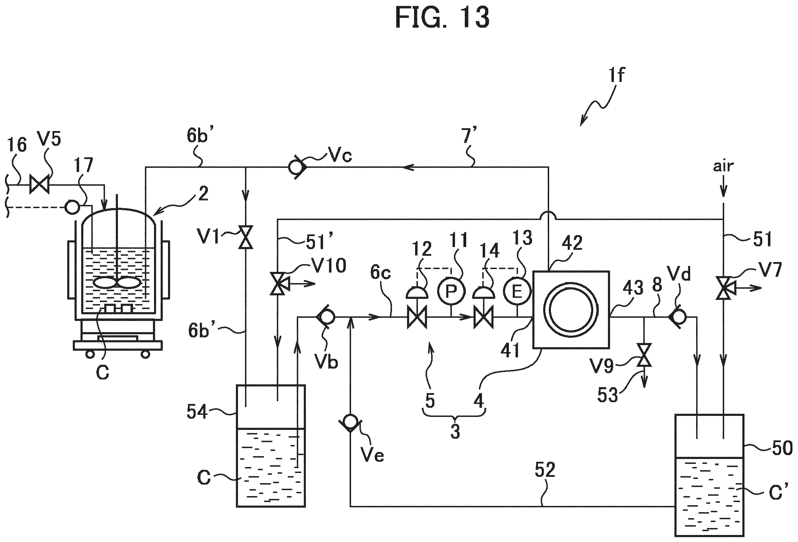

In the cell culture system 1e of FIG. 12, the liquid medium of the culture tank 2 is once housed in the pressurizing container 54 and then separated by the hydrodynamic separation device 4. That is, the flow of the liquid medium in the supply path 6b and the flow of the liquid medium in the reflux path 7 are alternately performed. Therefore, the supply path 6b and the reflux path 7 can be used by integrating their ends on the culture tank side into one. FIG. 13 shows an embodiment in which the end of the reflux path 7 on the culture tank side is integrated with the end of the supply path 6b in the cell culture system of FIG. 12.

In the cell culture system 1f of FIG. 13, an on-off valve V1 is installed, instead of a check valve, in the supply path 6b' that supplies the liquid medium C from the culture tank 2 to the pressurizing container 50. The liquid medium C of the pressurizing container 54 is separated by the hydrodynamic separation device 4 in the same manner as described above, and the liquid medium containing the relatively large cells concentrated is discharged from the outlet 42. One end of the reflux path 7' for sending the liquid medium to the culture tank 2 is connected to the outlet 42, and a check valve Vc is installed. Since the other end of the reflux path 7', that is, the end on the exit side is connected near the entrance of the supply path 6b', the entrance of the supply path 6b' can also be used as the exit of the reflux path 7'.

When the separation process is stopped in the cell culture system, the liquid medium tends to stay in the pipes constituting the supply path for sucking the liquid medium from the culture tank and the reflux path for returning the liquid medium from the hydrodynamic separation device 4. The cells trapped in the pipe cannot breath, and if the cells are returned from the flow path to the culture tank by resuming the separation process, the proliferation may be adversely affected. In this regard, in the cell culture system 1f of FIG. 13, the operation is stopped by the return from the reflux path 7' to the culture tank 2, and the on-off valve V1 and the check valve Vc of the supply path 6' and the reflux path 7' prevent the backflow of the liquid medium from the culture tank 2. Therefore, it is difficult for the liquid medium to stay in the pipe after the process is stopped. Since it is the same as the cell culture system of FIG. 12 except that the supply path and the reflux path are integrated, description thereof will be omitted and the above will be referred to.

In this way, utilizing the hydrodynamic separation technique, relatively large cells can be selectively concentrated and separated from the liquid medium being cultured. Therefore, by refluxing this together with a new liquid medium to the culture tank, cell culture can be continued while maintaining high proliferative power. The hydrodynamic separation can promote the concentration separation of cells much more efficiently than the centrifugation, and can concentrate and separate the cells with a high viability without damaging the cells as compared with the filter separation and the like. The fraction containing relatively small cells that have been separated and removed are purified after removing unnecessary substances such as cell debris, using a filter or the like. As a result, useful components can be recovered. In the hydrodynamic separation, the liquid medium is supplied to the curved flow channel at a relatively high speed, so that the cell culture system of the present disclosure has a high processing capacity for concentrating and separating cells and is sufficiently applicable to cell culture on a product manufacturing scale.

### Example 1

### <Batch culture>

Amino acid (L-alanyl-L-glutamine) and antibiotics (penicillin, streptomycin, amphotericin B) were added in appropriate amounts to 1.5 L of liquid medium (manufactured by GE Healthcare, product name: SH30934.01 HyCell CHO Medium). CHO cells (Chinese hamster ovary cells) were cultured in the liquid medium maintained at a temperature of 36.5 to 37.0°C. During culturing, the pH of the liquid medium was controlled so that the pH did not fall below 6.70.

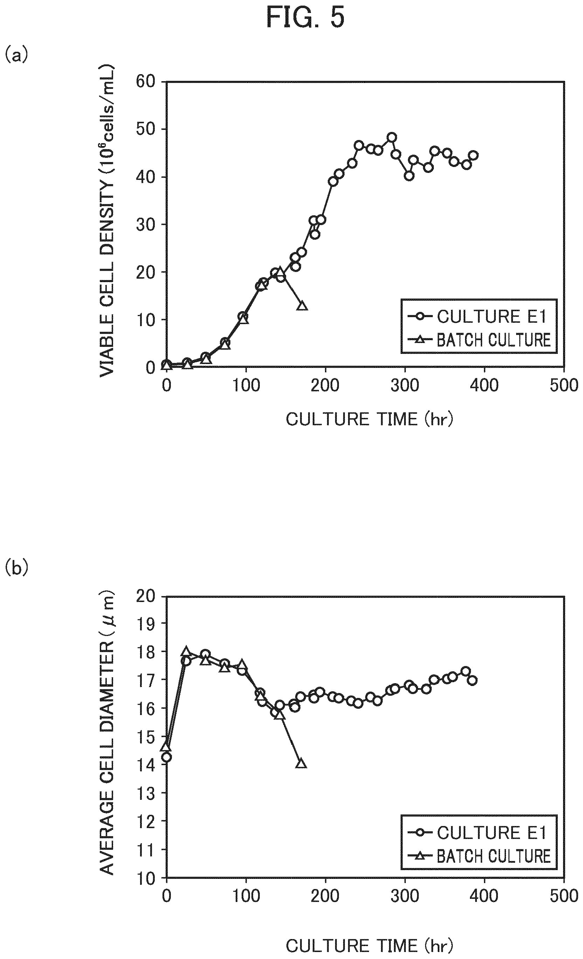

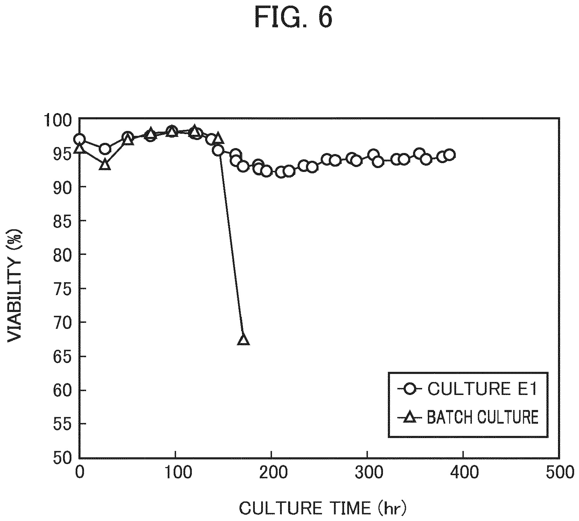

Sampling was performed during the culturing, and the viable cell density (×10⁶ cells/mL) and the average cell diameter (µm) were measured using a cell measuring device (Beckman Coulter, product name: Vi-Cell). The results are shown in the graphs of FIG. 5(a) and FIG. 5(b). The cell viability during this period is shown in the graph of FIG. 6.

### <Culture E1>

A flat plate-shaped resin molded body in which an arc-shaped curved flow channel was formed was produced by imitating the particle separator described in Patent Literature 2 described above. Using this molded body as a flow channel unit, a hydrodynamic separation device was constructed. Using gas pumping as the pump 10 for pumping the liquid medium, the cell culture system 1 of FIG. 1 was constructed with use of the hydrodynamic separation device, and the above-mentioned batch culture was carried out in the culture tank 2 for 100 hours.

After that, a predetermined amount of liquid medium was withdrawn from the culture tank in one separation treatment, and the liquid medium was pressure-fed to the hydrodynamic separation device at a pressure of 0.28 to 0.36 MPa (Dean number: about 47 to 70). The outer circumferential fraction discharged from the hydrodynamic separation device was returned to the culture tank, and the same amount of new liquid medium as the inner circumferential fraction was added. While repeating this separation process at regular time intervals so that the daily medium exchange rate was in the range of 0.5 to 1.4, the culture was continued. During this period, sampling was performed to measure the viable cell density (×10⁶ cells/mL) and the average cell diameter (µm). The results are shown in the graphs of FIG. 5(a) and FIG. 5(b). The cell viability during this period is shown in the graph of FIG. 6.

From FIG. 5 and FIG. 6, it can be seen that, when the culture time in batch culture exceeds 110 hours, cell proliferation becomes difficult due to lack of nutrients or poisoning of metabolites, and the viability is significantly reduced. On the other hand, when the liquid medium is extracted and relatively large cells are concentrated and separated, which are supplied to the culture tank together with the new liquid medium as in the above-mentioned culture E1, the cell proliferation proceeds even if the culture time exceeds 110 hours. Therefore, it can be seen that the culture can be continued for 300 hours or more. Further, the cultured cells maintain a cell diameter of 16 µm or more, and it is thus understood that relatively large cells are preferably separated and concentrated in the hydrodynamic separation device.

### Example 2

### <Cell separation test>

Cell culture was carried out for 100 hours using the same liquid medium and CHO cells as in Example 1. Using this liquid medium as a stock solution, the cell viability (ratio of living cells in all cells) was measured using the cell measuring device (Beckman Coulter, product name: Vi-Cell), and the result was 92.9 %. Moreover, when the cell concentration was measured, the total cell concentration was 2.86 ×10⁶ cells/mL, the viable cell concentration was 2.66 ×10⁶ cells/mL, and the average cell diameter was 14.87 µm.

Using a plunger pump, the above stock solution was pressure-fed to the hydrodynamic separation device of Example 1 at a pressure of 0.25 MPa (Dean number: 78) to divide into the outer circumferential fraction and the inner circumferential fraction.

Similarly, the cell concentration, average cell diameter, and cell viability were measured for the fraction on the inner circumferential side. As a result, the total cell concentration was 0.25 ×10⁶ cells/mL, the viable cell concentration was 0.18 ×10⁶ cells/mL, and the average cell diameter was 11.46 µm. The cell viability was 70.0 %.

When the fraction on the outer circumferential side was also measured in the same manner, the total cell concentration was 5.72 ×10⁶ cells/mL, the viable cell concentration was 5.37 ×10⁶ cells/mL, and the average cell diameter was 14.96 µm. The cell viability was 93.8 %.

From the above results, first, when comparing the cell concentrations, it is clear that the distribution of cells shifts to the outer circumferential side as the liquid medium flows through the curved flow channel. Further, from the comparison of average cell diameters, it is clear that the inner circumferential fraction contains relatively small cells (cell diameter: 11.46 µm), and relatively large cells (cell diameter: 14.96 µm) are concentrated in the outer circumferential fraction. Therefore, it is understood that relatively large cells can be selectively separated at a high concentration, by dividing the liquid medium into two fractions of the outer circumferential side and the inner circumferential side. Furthermore, since the viability in the fraction on the inner circumferential side is low, it can be seen that, by removal of relatively small cells, dead cells can be removed and a liquid medium containing a group of cells having a higher viability than the stock solution is obtained.

### Example 3

### <Effect of pressure environment on cells>

The following test system was constructed to investigate the effect of the pressure environment on cells in the hydrodynamic separation device. A pressure-resistant tank and the inlet of the hydrodynamic separation device were connected by piping via a one-way valve and a syringe pump, and two outlets of the hydrodynamic separation device and a pressure-resistant tank were connected by a Y-shaped pipe so that the two fractions discharged from the hydrodynamic separation device would both return to the pressure-resistant tank. Pressure gauges for measuring the supply pressure and outlet pressure of the liquid medium in the hydrodynamic separation device were provided in the piping and the Y-shaped pipe.

Cell culture was carried out for 100 hours using the same liquid medium and CHO cells as in Example 1, and the liquid medium after the culturing was filled in a pressure-resistant tank together with pressurized air of about 0.3 MPa. Then the following tests T1 to T10 were performed. Before and after the test, the viability of cells contained in the liquid medium was measured using a cell measuring device (Beckman Coulter, product name: Vi-Cell). The results are shown in Table 1. In the following tests, three types of curved flow channels (L1 to L3, having the same width but different height or length) including the curved flow channel of Example 1 were prepared, and a hydrodynamic separation device having either of them was used.

### (Tests T1 to T8)

Using a syringe pump, the liquid medium contained in the pressure-resistant tank was pressure-fed to the hydrodynamic separation device to perform the separation process, while adjusting the supply pressure as shown in Table 1. The two fractions discharged from the hydrodynamic separation device were combined and returned to the pressure-resistant tank. This separation process was repeated and the liquid medium was subjected to 10 times of the separation process. Note that the pressure of the fractions discharged from the hydrodynamic separation device is released to the atmospheric pressure, and the pressure fluctuation in the hydrodynamic separation device is thus equal to the supply pressure (inlet pressure) pumped to the hydrodynamic separation device.

### (Tests T9 and T10)

In the above test system, additionally, a pressure-regulating valve was attached to the pipe connected to the two outlets of the hydrodynamic separation device to enable the adjustment of the outlet pressure of the factions discharged from the hydrodynamic separation device. In this test system, while adjusting the supply pressure and the outlet pressure as shown in table 1, the separation process was repeated in the same manner as in the tests T1 to T8 and the liquid medium was subjected to 10 times of separation process. The pressure fluctuation in the hydrodynamic separation device is the difference between the supply pressure (inlet pressure) pumped to the hydrodynamic separation device and the pressure adjusted (outlet pressure) by the pressure regulating valve at the outlet.

**[Table 1]**

| | Supply Pressure [MPa] | Outlet Pressure [MPa] | Pressure Fluctuation [MPa] | Used Channel | Cell Viability [%] | | |
|---|---|---|---|---|---|---|---|
| | | | | | Before Test | After Test | Difference |
| Test T1 | 0.22 | 0 | 0.22 | L1 | 96.7 | 96.4 | -0.3 |
| Test T2 | 0.26 | 0 | 0.26 | L2 | 98.6 | 98.3 | -0.3 |
| Test T3 | 0.33 | 0 | 0.33 | L1 | 97.3 | 97.4 | 0.1 |
| Test T4 | 0.33 | 0 | 0.33 | L1 | 97.9 | 97.7 | -0.2 |
| Test T5 | 0.40 | 0 | 0.40 | L1 | 99.0 | 98.8 | -0.2 |
| Test T6 | 0.50 | 0 | 0.50 | L1 | 98.0 | 97.1 | -0.9 |
| Test T7 | 0.51 | 0 | 0.51 | L3 | 98.9 | 97.8 | -1.1 |
| Test T8 | 0.60 | 0 | 0.60 | L1 | 98.4 | 95.2 | -3.2 |
| Test T9 | 0.60 | 0.29 | 0.31 | L1 | 97.9 | 97.9 | 0.4 |
| Test T10 | 0.31 | 0 | 0.31 | L1 | 97.9 | 97.9 | 0.3 |

In the measurement of cell viability, the range from -0.5 % to +0.5 % can be regarded as an error, so the decrease in viability at a supply pressure of 0.6 MPa in the test T6 is clearly a significant result. From the results of tests T1 to T8 in table 1, it is considered that the threshold value at which the decrease in viability can be suppressed is around 0.45 MPa. However, in the test T9, the cell viability has been maintained high even when the supply pressure was 0.6 MPa. From this result, it can be seen that the cause of the decrease in the cell viability is not the static pressure but the magnitude of pressure fluctuation.

### Industrial Applicability

The relatively large cells among the cultured cells can be selectively concentrated and separated to continue the culture. Therefore, it contributes to the improvement of economy and quality in the provision of products such as hormones, cytokines, enzymes, antibodies, and vaccines, by application to the manufacture of pharmaceuticals using biotechnology. It will be possible to promote the spread and generalization of medicines that are rare or expensive at present.

### Explanation of Reference

- 1, 1a, 1b, 1c, 1d, 1d', 1e, 1f: cell culture system
- 2: culture tank
- 3: cell separator
- 4, 4a: hydrodynamic separation device
- 5, 5a: liquid feeding unit
- 6, 6a, 6b, 6c, 6': supply path
- 7, 7a, 7': reflux path
- 8, 8', 8a: recovery path
- 9: medium supplement unit
- 10, 10a: pump
- 11, 11a: pressure gage
- 12, 12a: pressure-regulating valve
- 13, 13a: flow meter
- 14, 14a, 18: flow rate-adjusting valve
- 15: medium tank
- 16: replenishment path
- 17: liquid level meter
- 19: tubing pump
- 20, 20a, 20b: recovery tank
- 21: filter
- 41: inlet
- 42, 42a, 43, 43a: outlet
- 50: temporary container
- 51, 51': gas line
- 52: return path
- 53: discharge path
- 54: pressurizing container
- V1, V2, V3, V4, V5, V6, V8, V9, V11: on-off valve
- V7, V10: residual pressure release valve
- Va, Vb, Vc, Vd, Ve: check valve
- C, CO, C', C": liquid medium

## Claims

1. A cell culture system, comprising a culture tank that contains a liquid medium that cultures cells, and a cell separator, the cell separator comprising:
a hydrodynamic separation device having a curved flow channel having a rectangular cross-section, and separating relatively large cells from the cells contained in the liquid medium using a vortex flow generated by flow through the curved flow channel; and
a liquid feeding unit which flows the liquid medium through the hydrodynamic separation device in a pressure environment controlled so as to suppress decrease in cell viability caused by pressure fluctuation in the liquid medium during flowing through the hydrodynamic separation device.

2. The cell culture system according to claim 1, wherein the hydrodynamic separation device has a single inlet for taking in the liquid medium and at least two outlets for discharging the separated liquid medium, wherein a liquid medium containing the relatively large cells concentrated is discharged from one of the at least two outlets, and the rest of the liquid medium containing relatively small cells is discharged from the other of the at least two outlets.

3. The cell culture system according to claim 1 or 2, wherein the liquid feeding unit has an urging device that urges the liquid medium with a flow pressure for supplying the liquid medium to the hydrodynamic separation device.

4. The cell culture system according to any one of claims 1 to 3, wherein the liquid feeding unit has a pressure control mechanism that controls the pressure environment so that a pressure difference between the liquid medium introduced to the hydrodynamic separation device and the liquid medium derived from the hydrodynamic separation device is equal to or less than a predetermined value.

5. The cell culture system according to claim 4, wherein the pressure control mechanism comprises:
a pressure monitoring unit to monitor a pressure of the liquid medium supplied to the hydrodynamic separation device; and
a pressure adjusting member that adjusts the pressure of the liquid medium supplied to the hydrodynamic separation device, based on the pressure monitored by the pressure monitoring unit.

6. The cell culture system according to any one of claims 1 to 5, wherein the liquid feeding unit further has a flow rate-controlling mechanism for controlling a flow rate of the liquid medium supplied to the hydrodynamic separation device.

7. The cell culture system according to claim 6, wherein the flow rate-controlling mechanism comprises:
a flow meter to monitor the flow rate of the liquid medium supplied to the hydrodynamic separation device; and
a flow rate-adjusting member to adjust the flow rate of the liquid medium supplied to the hydrodynamic separation device, based on the flow rate monitored by the flow meter,
wherein the flow rate of the liquid medium supplied to the hydrodynamic separation device is adjusted according to a size of the cells to be separated.

8. The cell culture system according to any one of claims 1 to 7, further comprising:
a circulation system which returns a liquid medium containing the relatively large cells separated by the hydrodynamic separation device of the cell separator to the culture tank, to circulate the liquid medium between the culture tank and the cell separator.

9. The cell culture system according to claim 8, further comprising:
a medium supplement unit which replenishes the culture tank with a new liquid medium corresponding to an amount of the rest of the liquid medium.

10. The cell culture system according to claim 9, wherein the medium supplement unit comprises:
a monitor that monitors an amount of the new liquid medium replenished in the culture tank; and
a flow rate-adjusting member that adjusts a flow rate of the new liquid medium replenished to the culture tank, based on the amount monitored by the monitor.

11. A cell culture method, comprising:
a cell culture for culturing cells in a liquid medium; and
a cell separation for separating, from the liquid medium, a liquid medium containing relatively large cells,
the cell separation comprising:
a separation process to separates the medium containing the relatively large cells concentrated from the liquid medium containing the cells, by using a vortex flow generated by flow through a curved flow channel having a rectangular cross-section; and
a pressure control to control a pressure environment of the liquid medium that flows in the separation process, so as to suppress a decrease in cell viability caused by pressure fluctuation in the liquid medium while flowing through the curved flow channel.

12. The cell culture method according to claim 11, further comprising:
a circulation process which returns the liquid medium containing the relatively large cells separated by the separation process to the cell culture, and circulates the liquid medium between the cell culture and the separation process.

13. The cell culture method according to claim 12, further comprising:
a medium supplementation which replenishes the cell culture with a new liquid medium corresponding to an amount of the rest of the liquid medium containing relatively small cells separated in the separation process.

14. The cell culture system according to claim 2, wherein the cell separator further comprises:
a temporary container for accommodating the rest of the liquid medium discharged from the other outlet of the hydrodynamic separation device;
an additional hydrodynamic separation device; and
an additional liquid feeding unit which flows the liquid medium contained in the temporary container through the additional hydrodynamic separation device in a pressure environment controlled so as to suppress decrease in cell viability caused by pressure fluctuation in the liquid medium during flowing through the additional hydrodynamic separation device.

15. The cell culture system according to claim 14, wherein the additional liquid feeding unit has an urging device that urges the liquid medium with a flow pressure for supplying the liquid medium to the additional hydrodynamic separation device, and the urging device comprises:
a pump that urges the liquid medium at a connection path connecting the temporary container and the additional hydrodynamic separation device; or
a pressurizing device that pressurizes the inside of the temporary container and supplies the flow pressure to the contained liquid medium.

16. The cell culture system according to claim 2, wherein the cell separator further comprises:
a temporary container for accommodating the rest of the liquid medium discharged from the other outlet of the hydrodynamic separation device;
a return path for resupplying the rest of the liquid medium contained in the temporary container to the hydrodynamic separation device; and
a switching mechanism for switching between supply of the liquid medium from the culture tank to the hydrodynamic separation device and supply of the rest of the liquid medium from the return path to the hydrodynamic separation device,
wherein, by switching of the switching mechanism, the liquid medium of the culture tank and the rest of the liquid medium of the temporary container are alternately supplied to the hydrodynamic separation device.

17. The cell culture system according to claim 16, wherein the liquid feeding unit includes: a supply path connecting the culture tank and the hydrodynamic separation device; and an urging device that urges the liquid medium with a flow pressure for supplying the liquid medium to the hydrodynamic separation device, and the return path is connected to join the supply path,
wherein the urging device comprises:
a pump that urges the liquid medium at the supply path; or
a pressurizing container that temporarily stores the liquid medium in the supply path and pressurizes the stored liquid medium to apply the flow pressure.

18. The cell culture system according to claim 2, wherein the liquid feeding unit includes: a supply path connecting the culture tank and the hydrodynamic separation device; and a pressurizing container that temporarily stores the liquid medium in the supply path and pressurizes the stored liquid medium to apply a flow pressure for supplying the liquid medium to the hydrodynamic separation device, and wherein the cell separator further comprises:
a temporary container for accommodating the rest of the liquid medium discharged from the other outlet of the hydrodynamic separation device;
a return path that connects the downstream side of the pressurizing container in the supply path with the temporary container and resupplies the rest of the liquid medium contained in the temporary container to the hydrodynamic separation device; and
a switching mechanism for switching between supply of the liquid medium from the culture tank to the hydrodynamic separation device and supply of the rest of the liquid medium from the return path to the hydrodynamic separation device,
wherein, by switching the switching mechanism, the liquid medium of the culture tank and the rest of the liquid medium of the temporary container are alternately supplied to the hydrodynamic separation device.

19. The cell culture system according to claim 18, wherein the switching mechanism has an on-off valve or a check valve provided on each of the upstream and downstream sides of the pressuring container in the supply path, and on the return path.

20. The cell culture system according to claim 19, wherein the cell separator further comprises a reflux path to send the liquid medium containing the relatively large cells concentrated from one of the outlets of the hydrodynamic separation device to the culture tank, and
an exit-side end of the reflux path is connected near an entrance of the supply path so that the entrance of the supply path is also possibly used as exit of the reflux path.

21. A cell culture system, comprising a culture tank that contains a liquid medium that cultures cells, and a cell separator, the cell separator comprising:
a hydrodynamic separation device having a curved flow channel having a rectangular cross-section, to separate relatively large cells from the cells contained in the liquid medium, using a vortex flow generated by flow through the curved flow channel; and
a liquid feeding unit which flows the liquid medium through the hydrodynamic separation device so as to suppress decrease in cell viability in the liquid medium during flowing through the hydrodynamic separation device.
